# EUROPEAN PATENT SPECIFICATION

(11) **EP 1 956 903 B1**
(45) Date of publication and mention of the grant of the patent: **11.01.2017**
(21) Application number: 06830157.1
(22) Date of filing: 28.11.2006
(51) Int. Cl.: A61K 8/02, A61K 8/34, A61L 9/01, A61L 9/14, A61L 15/20, A61L 15/46, A61Q 15/00

(54) **USE OF AN ABSORPTION PRODUCT COMPRISING PENTANE-1, 5-DIOL AS DEODORANT**
VERWENDUNG EINES ABSORBIERENDEN ARTIKELS, DER PENTAN-1,5-DIOL ALS DEODORANT ENTHÄLT
UTILISATION D'UN ARTICLE ABSORBANT COMPRENANT DU PENTANE-1, 5-DIOL COMME DEODORANT

(30) Priority: 29.11.2005 SE 0502610; 29.11.2005 US 740299 P
(43) Date of publication of application: 20.08.2008
(73) Proprietor: Ambria Dermatology AB, 566 33 Habo (SE)
(72) Inventor: FAERGEMANN, Jan, S-413 19 Göteborg (SE); STERNER, Olov, S-212 42 Malmö (SE); HEDNER, Thomas, S-520 10 Gällstad (SE)
(74) Representative: Roos, Rikard
(86) International application number: PCT/EP2006/069007
(87) International publication number: WO 2007/063065

(56) References cited:
- EP-A1- 0 806 195
- WO-A-2004/112765
- DE-A1- 19 924 496
- GB-A- 1 472 536

## Description

### Field of the Invention

The present invention relates to the use of a composition to reduce and/or eliminate odour from a mammal, such as odour from urine, faeces, liquids from leg ulcers, decubital ulcers, blood and menstruation, said composition being applied to absorbent and/or sanitary products.

### Background of the Invention

Today, the human being is using a huge amount of disposable products, such as napkins and diapers in which these products upon use by the human being becomes contaminated with different body liquids, such as urine, faeces, liquids from leg ulcers, decubital ulcers, blood and perspiration. Thereby the product and the human being using said product begins to smell. When the smell/odour has occurred the only possibility to reduce and/or eliminate said odour is to remove and discard said disposable product. All the disposable products, which are discarded increases the pressure on the environment. This could mean that the human being, consuming a disposable product to absorb for example urine, may change the disposable product several times every day to eliminate that an odour occur, which is not very nice to the surroundings or the consumer itself. The same applies for older people in institutions as well as in hospitals. If a disposable product was available on the market which had new improved properties, such as having the ability to reduce and/or eliminate odour from human liquids, there should be an improvement for the human consumer as well as for the environment, which human consumer could feel safe that no odour should occur upon leakage of for example urine or perspiration. Also, the costs could be reduced for the consumer, since there would be no need of changing the disposable product as often.

Therefore there is a need of new disposable product that fulfil the demands of reducing and/or eliminating the odour of liquids from human beings, which disposable product thereby reduces the costs for the consumer as well as reduces the pressure on the environment from disposable products.

WO 02/064177 describes a disposable absorbent article, comprising an absorbent body, and a cover enclosing the same, which cover may be impregnated with 2-methyl-2,4-pentanediol or propylene glycol. The 2-methyl-2,4-pentanediol or propylene glycol is applied on the cover to improve the absorption in this hydrophobic layer. Thus, nothing is mentioned about the use of 2-methyl-2,4-pentanediol or propylene glycol to reduce odour and a second substance, lactic acid, is in fact added to reduce odour. In fact, 2-methyl-2,4-pentanediol is highly unsatisfactory in respect of providing an anti-odour effect, since this substance has a strong odour in it self. Propylene glycol has moderate activity as an antimicrobial agent and will therefore only be moderately effective in reducing odour.

WO 03/092757 discloses a flexible absorbent sheet material, which may comprise a plasticizer, such as 1,3-propanediol or 1,4-butanediol. Nothing is mentioned about using a diol for obtaining an anti-odour effect. Rather the mentioned diols are used only to alter the mechanical properties of said sheet. In fact, 1,4-butanediol is unsuitable for use in high yields in connection with the human body, since it metabolizes to gamma hydroxyl butyrate (GHB), which substance affects the human body in a very negative and toxic way. Both 1,3-propanediol and 1,4-butanediol are moderately active as antimicrobial agents, due to their somewhat short carbon chain length, and will therefore only be moderately effective in reducing odour.

EP 0044543 discloses a topical pharmaceutical formulation, comprising a water miscible polyhydric alcohol. In EP 0044543 propylene glycol, butane-1,3-diol, polyethylene glycol, and glycerol are preferred, where propylene glycol is especially preferred, as such polyhydric alcohol. EP 0044543 fails to disclose which polyhydric alcohols that may provide an anti-odour effect, whereby EP 0044543 naturally also fails to distinguish between different polyhydric alcohols in respect of their suitability as agents for obtaining an anti-odour effect. The polyhydric alcohols mentioned as preferred and especially preferred are only moderately active as antimicrobial agents, due to their somewhat short carbon chain length, and will therefore only be moderately effective in reducing odour.

JP 2003081801 discloses 1,2-alkanediols, such as 1,2-pentanediol, 1,2-hexanediol, and 1,2-octanediol, for preventing body odour. However, these diols are not very suitable for using as anti-odour agents, since they have strong odours in themselves. 1,2-pentanediol has for example an aromatic odour which affects the convenience of the consumer in a negative way. Also, 1,2-hexanediol, and 1,2-octanediol have low solubility in bodily fluids, such as urine. Also, these diols are costly, which will render the products obtained by using said diols expensive.

Other documents disclosing similar compositions for use as anti-odour agent are JP 19920319931 and JP 2001190647.

Hence, an improved way of obtaining an anti-odour effect in absorbent products would be advantageous and in particular a method to reduce and/or eliminate odour from a mammal, such as odour from urine, faeces, liquids from leg ulcers, decubital ulcers, blood and menstruation, which method not creates other odours or smells that may be of inconvenience for the consumer, which method is cost-effective, and which method not is in need of the use of other odour hiding substances, such as perfumes, that may lead to allergies and/or which method not involves a simultaneous colouring of products, would be advantageous. Also, products obtained by such method, and compositions used in such methods, would be advantageous.

### Summary of the Invention

Accordingly, the present invention preferably seeks to mitigate, alleviate or eliminate one or more of the above-identified deficiencies in the art and disadvantages singly or in any combination and solves at least the above mentioned problems by providing the use of a composition comprising pentane-1,5-diol to reduce and/or eliminate odour from a mammal, such as odour from urine, faeces, liquids from leg ulcers, decubital ulcers, blood and menstruation, an absorption product comprising pentane-1,5-diol to reduce and/or eliminate odour from a mammal, such as odour from urine, faeces, liquids from leg ulcers, decubital ulcers, blood and menstruation.

By the invention the human being using said composition do not suffer from the odour that a body liquid coming from a human being normally give rise to. The composition is integrated/applied onto an absorbent and/or sanitary article and thereby reduces and/or eliminates the odour from the mammal upon use of said article.

### Detailed description of the Invention

In the context of the present invention the following definitions apply:
The term "article" and "product" within the application is intended to mean the same.

The term "odour" is intended to mean any odour that can arise from body liquids coming from a mammal such as a human being. Examples of body liquids are such as urine, faeces, liquids from leg ulcers, decubital ulcers, blood and perspiration as well as menstruation.

The term "absorbent product" is intended to mean any product that can absorb human liquids, such as bandage or compresses.

The term "sanitary product" is intended to mean any product that can be used to, at least partly, absorb urine , menstruation and/or faeces. Examples are diapers, and tampons.

### Use of a diol to reduce odour

The inventors disclose the use of a composition comprising pentane-1,5-diol to reduce and/or eliminate odour from a mammal, such as odour from urine, faeces, liquids from leg ulcers, decubital ulcers, blood, menstruation and perspiration.

By the use of pentane-1,5-diol to reduce and/or eliminate odour several problems have been solved, such as the consumer do not suffer from the odour and there is no need of changing for example a diaper frequently and thereby the costs of using diapers are reduced.

The use of pentane-1,5-diol to reduce and/or eliminate odour from a mammal, such as odour from urine, faeces, liquids from leg ulcers, decubital ulcers, blood and perspiration, provides several advantages in respect the diols used in the prior art. Firstly, pentane-1,5-diol is odourless, which gives pentane-1,5-diol a great advantage compared to many other diols, especially in respect of providing an anti-odour effect. Thus, when pentane-1,5-diol is used to reduce and/or eliminate odour there is no need to hide its odour. This in turn gives rise to another advantage with the use of pentane-1,5-diol, since there is no need to use perfumes, that may result in allergic reactions in mammals getting in touch with it, and since pentane-1,5-diol not gives rise to such allergic reactions in itself. Pentane-1,5-diol is also an effective antimicrobial agent, killing or reducing the number of bacteria and fungi. It is more effective than several other diols. Furthermore, there is a large access to pentane-1,5-diol on the market, since pentane-1,5-diol is a residual product in the manufacturing process of hexanediol, which is a major constituent in the manufacturing of plastics. Also, since pentane-1,5-diol is colourless, there is no need for colouring or decolourizing a consumer product after the application of pentane-1,5-diol. Still another advantage of pentane-1,5-diol is the fact that it is proven to have a low toxicity and eco-toxicity, and that these are well established.

Furthermore, pentane-1,5-diol presents a solubility that is satisfactory in respect of being soluble in polar fluids, such as water, sweat, urine, blood, menstruation, faeces, liquids from leg ulcers, decubital ulcers, and perspiration and other bodily fluids. This is because the carbon chain has a length giving a satisfactory solubility, while still providing a good anti-odour effect.

Both the solubility in aqueous solutions, such as sweat, urine, blood, menstruation, feaces, liquids from leg ulcers, decubital ulcers, and perspiration and other bodily fluids, and the antimicrobial effect are, at least partly, dependent of the chain length of the diol. Short chain length provides the diol with good solubility, but also poorer antimicrobial effect. The opposite is true for longer chains to a certain extent. Consequently these effects have to balanced to each other, in terms of providing an efficient anti-odour control of bodily fluids. Except for being odourless, pentane-1,5-diol have a high antimicrobial effect and a satisfactory solubility.

Since pentane-1, 5-diol has the above mentioned positive characteristics regarding providing good anti-odour effect, pentane-1,5-diol may be used to alleviate the above mentioned drawbacks of other anti-odour agents, such as the drawback that the anti-odour agent has a strong odour in itself, by providing compositions comprising pentane-1,5-diol and other anti-odour agents, such as other diols. Therefore, in another embodiment of the present invention, a composition is provided that also comprises at least another diol having the general structure of (CH₂)ₙH₂O₂, wherein n represents the number of CH₂. For example n is from 3 to 10, such as 3, 4, 5, 6, 7, 8, 9 or 10. Accordingly, the diol(s) may have hydroxyl groups on the diols, which are connected to different carbon atoms. Examples of diols are propane-1,2-diol, propane-1,3-diol, butane-1,2-diol, butane-1,3-diol, butane-1,4-diol, 2-methylpropane-1,2-diol, 2-methylpropane -1,3-diol, pentane-1,2-diol, pentane-1,3-diol, pentane-1,4-diol, 2-methylpentane-2,4-diol, pentane-2,3-diol, pentane-2,4-diol, hexane-1,2-diol, hexane-1,3-diol, hexane-1,4-diol, hexane-1,5-diol, hexane-1,6-diol, hexane-2,3-diol, hexane-2,4-diol, hexane-2,5-diol, hexane-3,4-diol, heptane-1,2-diol, heptane-1,3-diol, heptane-1,4-diol, heptane-1,5-diol, heptane-1,6-diol, heptane-1,7-diol, heptane-2,3-diol, heptane-2,4-diol, heptane-2,5-diol, heptane-2,6-diol, heptane-3,4-diol, heptane-3,5-diol, octane-1,2-diol, octane-1,3-diol, octane-1,4-diol, octane-1,5-diol, octane-1,6-diol, octane-1,7-diol, octane-1,8-diol, octane-2,3-diol, octane-2,4-diol, octane-2,5-diol, octane-2,6-diol, octane-2,7-diol, octane-3,4-diol, octane-3,5-diol, octane-3,6-diol and octane-4,5-diol. There may also be a mixture of different diols, such as 2, 3, 4 or 5 different diols, in combination with pentane-1,5-diol.

In still another embodiment a composition is provided, which composition comprises pentane-1,5-diol and at least one of the diols selected from the group consisting of propane-1,2-diol, propane-1,3-diol, butane-1,3-diol, pentane-1,3-diol, and pentane-1,4-diol. Propane-1,2-diol, propane-1,3-diol, butane-1,3-diol, pentane-1,3-diol, and pentane-1,4-diol also have a good anti-odour effect, and do not present as strong and/or unpleasant odours, like for example pentane-1,2-diol, and do not result in toxic metabolic residual products, like for example butane-1,4-diol.

In still another embodiment a composition is provided, which composition comprises pentane-1,5-diol and propane-1,2-diol and/or butane-1,3-diol. Propane-1,2-diol and butane-1,3-diol have a greater solubility than pentane-1,5-diol in urine, menstruation, faeces, liquids from leg ulcers, decubital ulcers, blood and perspiration, and a good anti-odour effect, while not presenting very strong odour in themselves. Thus, a composition comprising a combination of pentane-1,5-diol with these other diols has an improved solubility in comparison with a composition comprising only pentane-1,5-diol and less odour and enhanced antimicrobial activity compared to a composition comprising only propane-1,2-diol and/or butane-1,3-diol.

The stereochemistry of the diols is not important to the present invention, and enantiomers, diastereomers, tautomers and racemic mixtures of diols may all be used with good results. Indeed, the requirement that the diols be "different" should be understood to mean that they differ in the connectivity of the atoms (regioisomerism) and not in whether they are e.g. R/S or +/-.

The pentane-1,5-diol or pentane-1,5-diol and other diols may be present in an amount of from about 0.1 to about 99 % (v/v) depending on the article in which the diol(s) are used. Examples of amounts are 1, 10, 20, 30, 40, 50, 60, 70, 80, 90 or 99 %.

Accordingly, the pentane-1,5-diol or pentane-1,5-diol and other diols may be used together with other components, such as antibacterial, antifungal or antiviral components, such as antibiotics, antimycotics and antiviral agent. Examples are penicillins, cephalosporins, carbacephems, cephamycins, carbapenems, monobactams, aminoglycosides, glycopeptides, quinolones, tetracyclines, macrolides, fluoroquinolones, imidazoles, allylamines, acyclovir and vectavir. Examples are antiseptic agents include iodine, silver, copper, chlorhexidine, polyhexanide, alginate, zinc or zincoxide and other biguanides, acetic acid, and hydrogen peroxide. The pentane-1,5-diol or pentane-1,5-diol and other diols may also be used together with other compounds such as natural, synthetic or semisynthetic or natural identical, which provide smell to the product in which the diol is used. Examples are different perfumes, citrus, apple, and rose.

### Absorbent and/or sanitary product

The invention relates to an absorbent and/or sanitary product as defined above in which the product comprises a composition comprising pentane-1,5-diol. In another embodiment the invention also relates to an absorbent and/or sanitary product as defined above in which the product comprises a composition comprising pentane-1,5-diol and at least one other diol. This diol may comprise between 3 and 8 carbon atoms in a linear or branched chain. Accordingly, the diol(s) may have hydroxy groups of the diols that are connected to different carbon atoms. Examples of diols are propane-1,2-diol, propane-1,3-diol, butane-1,2-diol, butane-1,3-diol, butane-1,4-diol, 2-methylpropane-1,2-diol, 2-methylpropane -1,3-diol, pentane-1,2-diol, pentane-1,3-diol, pentane-1,4-diol, 2-methylpentane-2,4-diol, pentane-2,3-diol, pentane-2,4-diol, hexane-1,2-diol, hexane-1,3-diol, hexane-1,4-diol, hexane-1,5-diol, hexane-1,6-diol, hexane-2,3-diol, hexane-2,4-diol, hexane-2,5-diol, hexane-3,4-diol, heptane-1,2-diol, heptane-1,3-diol, heptane-1,4-diol, heptane-1,5-diol, heptane-1,6-diol, heptane-1,7-diol, heptane-2,3-diol, heptane-2,4-diol, heptane-2,5-diol, heptane-2,6-diol, heptane-3,4-diol, heptane-3,5-diol, octane-1,2-diol, octane-1,3-diol, octane-1,4-diol, octane-1,5-diol, octane-1,6-diol, octane-1,7-diol, octane-1,8-diol, octane-2,3-diol, octane-2,4-diol, octane-2,5-diol, octane-2,6-diol, octane-2,7-diol, octane-3,4-diol, octane-3,5-diol, octane-3,6-diol and octane-4,5-diol. There may also be a mixture of different diols, such as 2, 3, 4 or 5 different diols, together with pentane-1,5-diol. The stereochemistry of the diols is not important to the present invention, and enantiomers, diastereomers, tautomers and racemic mixtures of diols may all be used with good results. Indeed, the requirement that the diols be "different" should be understood to mean that they differ in the connectivity of the atoms (regioisomerism) and not in whether they are e.g. R/S or +/-.

The pentane-1,5-diol or pentane-1,5-diol and other diols may be present in an amount of from about 0.1 to about 99 % (v/v) depending on the article in which the diol(s) are used. Examples of amounts are 1, 10, 20, 30, 40, 50, 60, 70, 80, 90 or 99 %.

Accordingly the pentane-1,5-diol or pentane-1,5-diol and other diols may be used together with other components, such as antibacterial, antifungal and antivirial components, such as antibiotics, antimycotics and antiviral agent. Examples are penicillins, cephalosporins, carbacephems, cephamycins, carbapenems, monobactams, aminoglycosides, glycopeptides, quinolones, tetracyclines, macrolides, fluoroquinolones, imidazoles, allylamines, acyclovir and vectavir. Examples are antiseptic agents include iodine, silver, copper, chlorhexidine, polyhexanide, alginate, zinc or zincoxide and other biguanides, acetic acid, and hydrogen peroxide. The pentane-1,5-diol or pentane-1,5-diol and other diols may also be used together with other compounds such as natural, synthetic or semisynthetic or natural identical, which provide smell to the product in which the diol is used. Examples are citrus, apple, and rose.

The absorbent and/or sanitary article may be any absorbent or sanitary product as long as the product upon use provides the new properties of reducing and/or eliminating odour from mammalian liquids, such as human or animal liquids. Examples are disposable absorbing sheet, diaper, undergarment pad, tampons, bandages, compresses, face mask, sanitary napkin, tampon, panty shield, incontinence pad, incontinence device, surgical dressing, adhesive bandage, a paper product containing one or more paper webs, such as facial tissue, bath tissue, paper towels and napkins.

Following examples are intended to illustrate but not to limit the invention in any manner, shape, or form, either explicitly or implicitly.

### EXAMPLES

### Example 1

Active solutions containing 10 % (v/v) and 30 % (v/v) pentane-1.5-diol were sprayed onto a disposable napkin until the complete area of the napkin was moistened. The napkin was left at room temperature until the napkin was dry. The napkin was then used to remove urine from a human being. After removing the urine by the napkin it was found that there was no smell of urine from the napkin, which was nice for the consumer of the napkin. As a control was water used alone. The same amount of water and the active solution were used.

### Example 2

The same solution as in example 1 was sprayed onto a women diaper until the complete diaper was covered by the solution. The diaper was left in room temperature to dry. Upon use of the diaper by, a woman with urinary leakage problems, no smell could be detected even after a longer period of use.

### Example 3

Compresses were sprayed in the same way and with the same solutions as in Example 1.

### Example 4

Compresses were sprayed in the same way and with the same solutions as in Example 1. Such a compress can be used as a compress and applied onto an ulcer, such as leg ulcer or decubital ulcer.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. Use of an absorption product comprising pentane-1,5-diol to reduce and/or eliminate odour from a mammal, said odour being one of the following: odour from urine, menstruation, faeces, liquids from leg ulcers, decubital ulcers and blood.

2. Use according to claim 1, wherein said absorption product is used to reduce and/or eliminate odour from urine, liquids from leg ulcers and decubital ulcers.

3. Use according to any of claims 1-2, wherein said absorption product comprises at least another diol selected from the group consisting of propane-1,2-diol, propane-1,3-diol, butane-1,3-diol, pentane-1,3-diol and pentane-1,4-diol.

4. Use according to any of claims 1-3, wherein said absorption product comprises at least another diol selected from the group consisting of propane-1,2-diol and butane-1,3-diol.

5. Use according to any of claims 1-4, wherein said absorption product is a disposable adsorbing sheet, diaper, undergarment pad, tampons, bandages, compresses, sanitary napkin, panty shield, incontinence pad, incontinence device, surgical dressing, adhesive bandages.

6. Use according to any of claims 1-5, wherein said absorption product is a napkin, diaper and compresses.

7. Use according to any of claims 1-6, wherein said absorption product comprises a composition comprising 10 % (v/v) pentane-1,5-diol.

8. Use according to any of claims 1-6, wherein said absorption product comprises a composition comprising 20 % (v/v) pentane-1,5-diol.

9. Use according to any of claims 1-6, wherein said absorption product comprises a composition comprising 30 % (v/v) pentane-1,5-diol.

## Patentansprüche

1. Verwendung eines Absorptionsprodukts umfassend 1,5-Pentandiol, um Geruch eines Säugers zu verringern und/oder zu eliminieren, wobei der Geruch einer der folgenden ist: Geruch von Urin, Menstruation, Kot, Flüssigkeiten aus Beingeschwüren, Druckgeschwüren und Blut.

2. Verwendung nach Anspruch 1, wobei das Absorptionsprodukt verwendet wird, um Geruch von Urin, Flüssigkeiten aus Beingeschwüren und Druckgeschwüren zu verringern und/oder zu eliminieren.

3. Verwendung nach einem beliebigen der Ansprüche 1-2, wobei das Absorptionsprodukt mindestens ein anderes Diol umfasst, ausgewählt aus der Gruppe bestehend aus 1,2-Propandiol, 1,3-Propandiol, 1,3-Butandiol, 1,3-Pentandiol und 1,4-Pentandiol.

4. Verwendung nach einem beliebigen der Ansprüche 1-3, wobei das Absorptionsprodukt mindestens ein anderes Diol umfasst, ausgewählt aus der Gruppe bestehend aus 1,2-Propandiol und 1,3-Butandiol.

5. Verwendung nach einem beliebigen der Ansprüche 1-4, wobei das Absorptionsprodukt ein wegwerfbares adsorbierendes Tuch, eine Windel, eine Unterwäscheeinlage, Tampons, Verbände, Kompressen, eine Binde, eine Slipeinlage, eine Inkontinenzeinlage, eine Inkontinenzvorrichtung, ein Wundverband, ein Heftpflasterverband ist.

6. Verwendung nach einem beliebigen der Ansprüche 1-5, wobei das Absorptionsprodukt eine Einlage, eine Windel und Kompressen ist.

7. Verwendung nach einem beliebigen der Ansprüche 1-6, wobei das Absorptionsprodukt eine Zusammensetzung umfassend 10% (Vol./Vol.) 1,5-Pentandiol umfasst.

8. Verwendung nach einem beliebigen der Ansprüche 1-6, wobei das Absorptionsprodukt eine Zusammensetzung umfassend 20% (Vol./Vol.) 1,5-Pentandiol umfasst.

9. Verwendung nach einem beliebigen der Ansprüche 1-6, wobei das Absorptionsprodukt eine Zusammensetzung umfassend 30% (Vol./Vol.) 1,5-Pentandiol umfasst.

## Revendications

1. Utilisation d'un produit d'absorption comprenant du pentane-1,5-diol pour réduire et/ou éliminer l'odeur d'un mammifère, ladite odeur étant l'une des suivantes : une odeur d'urine, de menstruations, de fèces, de liquides d'ulcères de jambe, d'ulcères de décubitus et de sang.

2. Utilisation selon la revendication 1, dans laquelle ledit produit d'absorption est utilisé pour réduire et/ou éliminer une odeur d'urine, de liquides d'ulcères de jambe et d'ulcères de décubitus.

3. Utilisation selon l'une quelconque des revendications 1 à 2, dans laquelle ledit produit d'absorption comprend au moins un autre diol choisi dans le groupe constitué par le propane-1,2-diol, le propane-1,3-diol, le butane-1,3-diol, le pentane-1,3-diol et le pentane-1,4-diol.

4. Utilisation selon l'une quelconque des revendications 1 à 3, dans laquelle ledit produit d'absorption comprend au moins un autre diol choisi dans le groupe constitué par le propane-1,2-diol et le butane-1,3-diol.

5. Utilisation selon l'une quelconque des revendications 1 à 4, dans laquelle ledit produit d'absorption est une feuille adsorbante jetable, une couche, un tampon de sous-vêtement, des tampons, des bandages, des compresses, une serviette hygiénique, un protège-slip, un tampon d'incontinence, un dispositif d'incontinence, un pansement chirurgical, des bandages adhésifs.

6. Utilisation selon l'une quelconque des revendications 1 à 5, dans laquelle ledit produit d'absorption est une serviette, une couche et des compresses.

7. Utilisation selon l'une quelconque des revendications 1 à 6, dans laquelle ledit produit d'absorption comprend une composition comprenant 10 % (v/v) de pentane-1,5-diol.

8. Utilisation selon l'une quelconque des revendications 1 à 6, dans laquelle ledit produit d'absorption comprend une composition comprenant 20 % (v/v) de pentane-1,5-diol.

9. Utilisation selon l'une quelconque des revendications 1 à 6, dans laquelle ledit produit d'absorption comprend une composition comprenant 30 % (v/v) de pentane-1,5-diol.
